Europäisches Patentamt

(19) European Patent Office

Office européen des brevets

(11) EP 0 892 268 A1

(12) EUROPEAN PATENT APPLICATION

(43) Date of publication:
20.01.1999 Bulletin 1999/03

(51) Int. Cl.$^6$: G01N 31/16, G01N 33/28

(21) Application number: 98111303.8

(22) Date of filing: 19.06.1998

(84) Designated Contracting States:
AT BE CH CY DE DK ES FI FR GB GR IE IT LI LU MC NL PT SE
Designated Extension States:
AL LT LV MK RO SI

(30) Priority: 18.07.1997 IT MI971703

(71) Applicant: ENITECNOLOGIE S.p.A.
20097 S. Donato Milanese (Milano) (IT)

(72) Inventors:
• Pirovano, Carmen
20097 S. Donato Milanese (MI) (IT)

• Radaelli, Enrico
20047 Brugherio (Milano) (IT)
• Soprani, Massimo
13011 Borgosesia (Vercelli) (IT)
• Correra, Sebastiano
20097 S. Donato Milanese (Milano) (IT)

(74) Representative:
Gennari, Marco, Dr.
c/o EniTecnologie S.p.A.,
BREL,
Via F. Maritano, 26
20097 San Donato Milanese, Milano (IT)

(54) **Method for determining the flocculation of asphaltenes in oil**

(57) In the conductometric titration of oil samples with a flocculating agent, eg. cetane, in order to determine the flocculation threshold of asphaltenes in oil, the equivalence point is detected by monitoring the sign of the first differential of the titration curve.

fig 1

$y = 0,2218x + 0,2082$

EP 0 892 268 A1

## Description

The present invention relates to a method for determining the flocculation threshold of asphaltenes in oil.

The term oil refers to crude petroleum, the atmospheric or vacuum distillation residues coming from primary distillation and/or conversion and each petroleum product obtained by dilution of these oils with aromatic and/or paraffinic solvents.

The separation of asphaltenes is normally an undesired phenomenum caused by negative effects which are produced whenever it occurs. In fact, the precipitation and deposition following the destabilization of asphaltenes, cause:

- a reduction in the production of crude oil (deposition in the well, extraction tubing);
- a reduction in the potential yield of the refinery conversion processes;
- an increase in the maintenance costs ("fouling" of the transfer lines of the conversion reactors, storage tanks).

Destabilization can be caused by variations in temperature, pressure and the system composition; it is often a consequence of the simultaneous variation in the three parameters creating considerable difficulties in the interpretation of the mechanisms which form the basis of the phenomenum.

The conductimetric titration technique, as a method for determining the flocculation threshold of asphaltenes, was proposed in 1993 by Norsk Hydro (EP-544354). It is based on the principle according to which the electrical conductivity of a petroleum sample, however small, is due to the presence of asphaltene species which are electrically charged. Starting from this hypothesis, the precipitation of asphaltenes can be followed by analyzing the trend of the conductivity and/or electrical capacity of the petroleum product upon the addition of a titrant (paraffin) which acts as a precipitant with respect to asphaltenes.

The determination method claimed in EP-544354 comprises the following steps:

- transfer of a part of the petroleum product to a measurement and survey cell of the initial conductivity and/or initial capacity of the petroleum product;
- addition of an amount of titrant to the petroleum product, transfer to the measurement cell of a part of the titrated petroleum product and survey of the present conductivity and present capacity of said petroleum product;
- comparison of the conductivity and/or capacity values and control of the possible variation in the conductivity and/or capacity.

With the process claimed in this patent application, it is possible to identify the flocculation threshold as maximum value of the trend of the normalized specific conductivity with respect to the weight fraction of the titrant.

The normalized specific conductivity is defined as a ratio of the specific conductivity of the petroleum product on the weight fraction of the oil, i.e. on the quantity of oil in solution (oil + titrant).

According to the procedure described, the presence of a maximum in the conductivity trend is due to the presence of two conflicting effects caused by the titrant, which are the decrease in viscosity of the medium and the precipitating action with respect to the asphaltenes.

In addition to these two effects, there is the diluent effect of the titrant which, having an electrical conductivity generally lower than that of the petroleum sample, also causes a progressive reduction in the background signal.

Upon the first additions of the titrant, there is an increase in the conductivity of the system due to the more or less sensible decrease in the viscosity of the medium and consequent increase in the mobility of the charged species (asphaltenes).

With the subsequent additions, as the viscosity reduction effect becomes less significant, the subtraction mechanism of the charged species on the part of the titrant becomes prevalent and the conductivity starts to decrease.

The flocculation threshold point is made to coincide with the maximum of the normalized specific conductivity curve.

This procedure cannot be applied in the case of diluted petroleum systems, i.e. with systems which have a viscosity at 25°C of less than 10 cSt.

With diluted petroleum systems the reduction effect of the viscosity becomes exhausted at the first additions of titrant and the dilution effects which cause a decrease in the conductivity of the system prevail, so that the flocculation threshold cannot coincide with the normalized specific conductivity maximum.

We have now found a method which uses an algorithm that allows the flocculation threshold to be determined also on diluted systems.

The method for determining the flocculation threshold of asphaltenes in oil, optionally diluted, of the present invention, essentially comprises the following steps:

- transfer of a part of oil as such or diluted to a measurement and survey cell of the initial electrical resistance of the transferred oil;
- addition, at specific and constant time intervals, of equal amounts of titrant to the transferred oil or of constantly increasing amounts of titrant to equal amounts of non-titrated oil as such or diluted;
- transfer of the titrated oil to the measurement cell after each addition of titrant and survey of the electrical resistance of said titrated oil, at least twice, in the tine range falling between one addition of titrant and the following one;
- analysis of the resistance values observed in this time interval,
  characterized in that after each addition of titrant this analysis is carried out by means of the following steps:
- calculation of the angular parameter m defined by the following equation:

$$m = \frac{y_2 - y_1}{t_2 - t_1}$$

wherein y is the resistance or any other electrical variable obtainable therefrom, t the survey time of the resistance or any other electrical variable obtainable therefrom, calculated starting from the beginning of the titration, $(t_1, y_1)$ and $(t_2, y_2)$ are pairs of values in succession;
- calculation of the average angular parameter as average of the angular parameters calculated above,
  and characterized in that the determination of the threshold value is carried out as follows:

- sequential comparison of the average angular parameter signs calculated up to the identification of the sign change of one angular parameter with respect to the previous one;
- determination of the threshold value by means of the total quantity of titrant added in correspondence with the addition number immediately before that in which the sign change of the average angular parameter had been verified.

The angular parameters calculated for each addition of titrant are preferably in a number ranging from 4 to 6.

The titrant used is preferably selected from paraffins: in particular pentane, n-heptane and n-hexadecane (cetane) are recommended. The volume of titrant added for each addition must be constant and depends on the quantity of sample analyzed: for example, for a quantity of sample of less than 100 g a quantity of titrant at fixed volumes of 0.5 to 5 ml can be added.

The addition of the titrant can be carried out directly in the measurement cell or outside the cell in an appropriate tank.

The diluent of the oil can be an aromatic solvent or any compound which reduces the viscosity of the oil itself: in particular toluene can be used.

The time intervals falling between one addition of titrant and the subsequent one are constant as specified above and are preferably selected from 3 to 30 minutes, more preferably from 5 to 15 minutes.

EXAMPLE

The petroleum sample analyzed is a fluid fuel oil having a density at 15°C of 0.965 g/cm$^3$ and a viscosity at 25°C of 210 cSt, at 50°C of 49 cSt, at 100°C of 9.2 cSt with a content of asphaltenes/n-C$_7$ of 12.7% by weight (IP 143 Method) and a P-value of 1.3 (UNI 20011).

The conductimetric titrations were carried out both on the sample as such and on the sample diluted with toluene in ratios of 1/0.5 and 1/1.

The sample diluted in proportions of 1/0.5 has a density of 0.929 g/cm$^3$ and a viscosity at 25°C of 7.4 cSt, at 50°C of 4.0 cSt, at 100°C of 1.8 cSt, whereas the 1/1 diluted sample has a density of 0.910 g/cm$^3$ and a viscosity at 25°C of 2.9 cSt, at 50°C of 1.9 cSt, at 100°C of 1.1 cSt.

Experimental equipment and titration procedure

The titration was carried out using a closed circuit (loop) system in which the sample is circulated between the flask, where the mixture of petroleum sample with the precipitant (added by means of an external system) is formed and the electrochemical cell where the electrical resistance is measured.

The measurement loop consists of the following components:

- a three-necked flask, into which the starting solution is charged, maintained under constant stirring by magnetic stirring, and in which the subsequent additions of precipitant are carried out; two necks are used for the circulation of the solution (inlet, outlet), the third for the inlet of the precipitant.

- a recirculation pump which makes the solution circulate between the mixing flask and the conductimetric cell;
- a conductrimetric measurement cell suitable for operating with high electrical resistance systems;
- a dosing pump which feeds the precipitant at regular intervals into the mixing container.

Electrical resistance measurement system.

The conductivity measurements of the petroleum systems which are characterized by a high electrical resistance require a measurement cell with a low geometrical constant (l/S), which can be obtained by increasing the effective area of the electrodes and reducing their distance, in accordance with the following equation:

$$R = \rho * l/S$$

wherein    R is the resistance of the conductor (Ohm),
$\rho$ is the resistivity (Ohm*cm),
l/S is the geometrical constant of the system ($cm^{-1}$).

The most widely proposed geometry in these cases is the coaxial two cylinder geometry where the external surface of the internal cylinder and the internal surface of the external cylinder act as electrodes. Following this geometry, the measurement cell was constructed using two coaxial inox steel cylinders (AISI 316 L), 10 cm long and 0.1 cm apart. The surfaces which have the function of electrode were electrolytically lined with gold and were inserted inside a teflon casing equipped with two plugs with inlets for the feeding of the solution and the outlet of the electrical contacts.

The instrument used as impedance measurement system, consists of a FRA 1260 Impedance/Gain Phase Analyser frequency generator, connected to a potentiostat/galvanostat mod. 273 of EG&G, which can be run by computer with an IEEE488 card of GPIB. The measurement cell is electrically connected to the potentiostat/galvanostat.

The system measures the impedance, which is calculated as a ratio between the alternating voltage applied and the response current obtained by the same system, also taking into account the phase shift and amplitude variation which the current undergoes when it passes through the solution under examination.

Using appropriate algorithms, it is possible to trace from the impedance, the resistance (R) of the solution and from this other electrical variables as indicated hereunder.

Transformation of the resistance into specific conductivity ($\kappa$) and normalized specific conductivity ($\kappa_0$)

The specific conductivity ($\kappa$) is obtained starting from the resistance data as follows:

$$\kappa = l/R \times l/S$$

wherein l/S is the cell constant in $cm^{-1}$ and R in $\Omega$

The cell constant was determined by measuring the resistance of the measurement cell completely filled with a solution of KCl $10^{-3}$ M with a known conductivity (or measured with other equipment). The value of l/S is obtained from the ratio between the resistance measured and the specific conductivity of KCl. The normalized specific conductivity ($\kappa_0$) is obtained by dividing the specific conductivity by the fraction of oil in solution:

$$\kappa_0 = \kappa / f_{oil}$$

Data determination

In the case of titration of fuel oil as such, 40 g of sample were weighed and charged into a mixing flask immersed in a thermostat bath regulated at 21°C and the recirculation pump was activated so as to completely fill the electrochemical measurement cell. The precipitant used (cetane) was then added using an automatic pipet on which 50 additions of a volume of 0.5 ml every 20 minutes had been programmed.

The impedance measurer was connected to the electrochemical cell, set at a fixed frequency of 20 Hz and programmed to reveal the resistance of the system at relatively short time intervals (generally in the range of tens of seconds).

The fixed frequency value depends on the sample being tested and is established on the basis of impedance measurements carried out within a wide range of frequencies (from several Hz to MHz).

The resistance data measured were processed with a suitable calculation program which operates after each addition as follows:

it eliminates the first resistance value revealed;

it calculates the angular parameter between each pair of resistances revealed;

it calculates the average of the angular parameters;

it compares the average angular parameter sign with the previous one;

it selects the angular parameter with a different sign with respect to the previous one;

it reveals the corresponding titration time;

it converts the titration time into the number of additions and consequently into the total quantity of precipitant added (in grams) per quantity of oil (in grams).

This ratio indicated with mp/mo corresponds to the flocculation threshold of asphaltenes.

Table I indicates for the fuel oil, the resistance values revealed close to the threshold, the corresponding times and the average angular parameters evaluated as described above.

As can be seen in Table 1, the sign change of the average angular parameter takes place in the 23rd addition: consequently the beginning of the flocculation of asphaltenes occurs at the 22nd addition. Knowing that the volume of each addition was 0.5 ml and that the quantity of sample weighed was 40 g, the flocculation threshold is

$$\frac{22 \times 0.5 \text{ ml} \times 0.773 \text{ g/mg}}{40 \text{ g}} = 0.212$$

wherein 0.773 g/ml is the density of the cetane measured at 15°C.

This value represents the minimum quantity in grams of precipitant to be added to 1 g of oil to make the asphaltenes precipitate.

When the flocculation threshold is expressed as the volume of cetane to be added to 1 g of sample, a comparison can be made with the P-Value determined with the procedure described by regulation UNI 20011.

In fact the P-Value definition is the following:

$$\text{P-Value} = 1 + (V_{cetane} \text{ (ml)}/ P_{oil} \text{ (g)})$$

wherein V is the maximum volume of cetane which can be added to the sample without there being any separation of the asphaltenes.

At the 22nd addition the quantity of cetane at the threshold is equal to 11 ml.

With the values obtained:

$$\text{P-Value} = 1.28$$

a value close to 1.3 of the oil determined with the method contained in regulation UNI 20011.

24.6 g of fuel oil dissolved in 12.4 g of toluene were titrated with the same procedure. Table II indicates the resistance data close to the flocculation threshold which in this case takes place at the 21st addition.

Using the above procedure the threshold in this case is:

$$\frac{21 \times 0.5 \text{ ml} \times 0.773 \text{ g/ml}}{24.6 \text{ g}} = 0.320$$

Finally, with the same procedure 18 g of fuel oil dissolved in 18 g of toluene were titrated and the results indicated in Table II were obtained around the flocculation threshold (20th addition).

Using the above procedure the threshold in this case is:

$$\frac{20 \times 0.5 \text{ ml} \times 0.773 \text{ g/ml}}{18.0 \text{ g}} = 0.430$$

At this point, in accordance with literature (see Hotier G., Robin M. - Action de divers diluants sur les produits pétroliers lourds: mesure, interpretation et prevision de la flocculation des asphaltènes. Revue de l'IFP, vol. 38, Nr. 1, 1983), by indicating on a graph the flocculation thresholds obtained in relation to the degree of oil dilution, which in the first case is equal to 0.5 and in the second to 1, and extrapolating to zero (dilution degree nil), the flocculation threshold of the product as such is obtained.

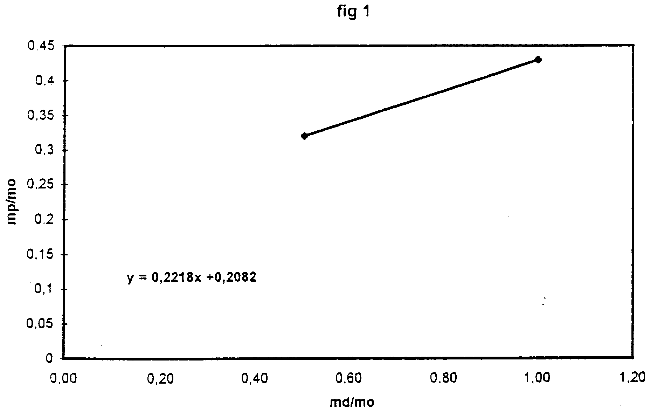

Figure 1 shows the flocculation threshold of the product as such obtained by the above extrapolation.

As can be seen from the line equation ($y = 0.2218x+0.2082$), the extrapolation at dilution degree nil is equal to 0.2082 close to the 0.21 obtained experimentally with the procedure of the invention.

Table 1

| | Nr. addition/Nr. record. | Time (minutes) | R (Ohm) | m (Ohm/min.) | $m_{average}$ |
|---|---|---|---|---|---|
| 21 | 2 | 100.6 | 908372 | | |
| | 3 | 101.3 | 908517 | 207.14 | |
| | 4 | 102.0 | 908089 | | |
| | 5 | 102.7 | 908141 | 74.28 | |
| | 6 | 103.4 | 908455 | | |
| | 7 | 104.1 | 907780 | -964.29 | -227.6 |
| 22 | 2 | 105.5 | 906741 | | |
| | 3 | 106.2 | 906471 | -385.7 | |
| | 4 | 106.9 | 906799 | | |
| | 5 | 107.6 | 906488 | -444.29 | |
| | 6 | 108.3 | 906324 | | |
| | 7 | 109.0 | 906034 | -414.28 | -414.8 |
| 23 | 2 | 110.4 | 905632 | | |
| | 3 | 111.1 | 905086 | -780.00 | |
| | 4 | 111.8 | 905094 | | |
| | 5 | 112.5 | 905316 | 317.14 | |
| | 6 | 113.2 | 905351 | | |
| | 7 | 113.9 | 905741 | 557.14 | 31.4 |
| 24 | 2 | 115.3 | 905340 | | |
| | 3 | 116.0 | 905206 | -191.43 | |
| | 4 | 116.7 | 904700 | | |
| | 5 | 117.4 | 904779 | 112.85 | |
| | 6 | 118.1 | 905259 | | |
| | 7 | 118.8 | 905370 | 158.57 | 26.7 |
| 25 | 2 | 120.2 | 905421 | | |
| | 3 | 120.9 | 905106 | -450.00 | |
| | 4 | 121.6 | 904617 | | |
| | 5 | 122.3 | 905118 | 715.71 | |
| | 6 | 123.0 | 905131 | | |
| | 7 | 123.7 | 905369 | 340.00 | 201.9 |

Table 2

| Nr. addition/Nr. record. | | Time (minutes) | R (Ohm) | m (Ohm/min.) | $m_{average}$ |
|---|---|---|---|---|---|
| 20 | 2 | 110.9 | 133889 | | |
| | 3 | 111.6 | 133858 | -44.28 | |
| | 4 | 112.3 | 133807 | | |
| | 5 | 113.0 | 133864 | 81.43 | |
| | 6 | 113.7 | 133895 | | |
| | 7 | 114.4 | 133806 | -127.14 | -30.0 |
| 21 | 2 | 115.6 | 133715 | | |
| | 3 | 116.3 | 133691 | -32.43 | |
| | 4 | 117.0 | 133737 | | |
| | 5 | 117.7 | 133752 | 21.42 | |
| | 6 | 118.4 | 133738 | | |
| | 7 | 119.1 | 133718 | -28.57 | -13.8 |
| 22 | 2 | 120.4 | 133571 | | |
| | 3 | 121.1 | 133579 | 11.43 | |
| | 4 | 121.8 | 133632 | | |
| | 5 | 122.5 | 133630 | -2.86 | |
| | 6 | 123.2 | 133763 | | |
| | 7 | 123.9 | 133761 | -2.86 | 1.9 |
| 23 | 2 | 125.3 | 134036 | | |
| | 3 | 126.0 | 134143 | 152.86 | |
| | 4 | 126.7 | 134332 | | |
| | 5 | 127.4 | 134411 | 112.86 | |
| | 6 | 128.1 | 134469 | | |
| | 7 | 128.8 | 134522 | 75.71 | 113.8 |
| 24 | 2 | 129.4 | 134744 | | |
| | 3 | 130.1 | 134868 | 177.14 | |
| | 4 | 130.8 | 134963 | | |
| | 5 | 131.5 | 135088 | 178.57 | |
| | 6 | 132.2 | 135213 | | |
| | 7 | 132.9 | 135250 | 52.85 | 136.19 |

Table 3

| | Nr. addition/Nr. record. | Time (minutes) | R (Ohm) | m (Ohm/min.) | $m_{average}$ |
|---|---|---|---|---|---|
| 19 | 2 | 95.8 | 131117 | | |
| | 3 | 96.5 | 131027 | -128.57 | |
| | 4 | 97.2 | 131037 | | |
| | 5 | 97.9 | 131057 | 28.57 | |
| | 6 | 98.6 | 131017 | | |
| | 7 | 99.3 | 130997 | -28.57 | -42.8 |
| 20 | 2 | 100.7 | 134247 | | |
| | 3 | 101.4 | 134127 | -172.43 | |
| | 4 | 102.1 | 134037 | | |
| | 5 | 102.8 | 133947 | -128.57 | |
| | 6 | 103.5 | 133957 | | |
| | 7 | 104.2 | 133927 | -42.85 | -114.3 |
| 21 | 2 | 105.6 | 136857 | | |
| | 3 | 106.3 | 136727 | -185.71 | |
| | 4 | 107.0 | 136747 | | |
| | 5 | 107.1 | 136867 | 171.43 | |
| | 6 | 108.4 | 136917 | | |
| | 7 | 109.1 | 136947 | 42.85 | 9.5 |
| 22 | 2 | 110.5 | 139668 | | |
| | 3 | 111.2 | 140118 | 642.86 | |
| | 4 | 111.9 | 140128 | | |
| | 5 | 112.6 | 140138 | 14.28 | |
| | 6 | 113.3 | 140088 | | |
| | 7 | 114.0 | 140098 | 14.28 | 233.8 |
| 23 | 2 | 115.4 | 142684 | | |
| | 3 | 116.1 | 143068 | 548.57 | |
| | 4 | 116.8 | 143038 | | |
| | 5 | 117.5 | 143058 | 28.57 | |
| | 6 | 118.2 | 142978 | | |
| | 7 | 118.9 | 142988 | 14.28 | 197.1 |

**Claims**

1.  A method for determining the flocculation threshold of asphaltenes in oil, optionally diluted, essentially comprising the following steps:

    -   transfer of a part of oil as such or diluted to a measurement and survey cell of the initial electrical resistance of the transferred oil;
    -   addition, at specific and constant time intervals, of equal amounts of titrant to the transferred oil or of constantly increasing amounts of titrant to equal amounts of non-titrated oil as such or diluted;

- transfer of the titrated oil to the measurement cell after each addition of titrant and survey of the electrical resistance of said titrated oil, at least twice, in the time range falling between one addition of titrant and the following one;
- analysis of the resistance values observed in this time interval,

characterized in that after each addition of titrant this analysis is carried out by means of the following steps:

- calculation of the angular parameter m defined by the following equation:

$$m = \frac{y_2 - y_1}{t_2 - t_1}$$

wherein y is the resistance or any other electrical variable obtainable therefrom, t the survey time of the resistance or any other electrical variable obtainable therefrom, calculated starting from the beginning of the titration, $(t_1, y_1)$ and $(t_2, y_2)$ are pairs of values in succession;

- calculation of the average angular parameter as average of the angular parameters calculated above,

and characterized in that the determination of the threshold value is carried out as follows:

- sequential comparison of the average angular parameter signs calculated up to the identification of the sign change of one angular parameter with respect to the previous one;
- determination of the threshold value by means of the total quantity of titrant added in correspondence with the addition number immediately before that in which the sign change of the average angular parameter has been verified.

2. The method according to claim 1, wherein the angular parameters calculated for each addition of titrant are in a number ranging from 4 to 6.

3. The method according to claim 1, wherein the titrant is a paraffin.

4. The method according to claim 3, wherein the paraffin is selected from pentane, n-heptane, n-hexadecane (cetane).

5. The method according to claim 1, wherein the oil diluent is toluene.

6. The method according to claim 1, wherein the time intervals falling between one addition of titrant and the subsequent one are between 3 and 30 minutes.

7. The method according to claim 6, wherein the time intervals are between 5 and 15 minutes.

fig 1

$$y = 0,2218x + 0,2082$$

**European Patent Office**

**EUROPEAN SEARCH REPORT**

Application Number

EP 98 11 1303

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,Y | EP 0 544 354 A (NORSK HYDRO TECHNOLOGY) 2 June 1993 * column 3, line 40 – column 6, line 9; figures 1,3 * | 1-7 | G01N31/16 G01N33/28 |
| Y | US 4 058 365 A (KROGH S-C) 15 November 1977 * column 1, line 33 – column 9, line 21; figure 1 * | 1-7 | |
| A | US 3 870 466 A (RELLSTAB W ET AL) 11 March 1975 * column 1, line 60 – column 2, line 45; column 3, line 15 – column 5, line 34; column 6, line 20 – column 8, line 42; figures 1,3,5,6 * | 1,2,6,7 | |
| D,A | HOTIER G ET AL: "Action de divers diluants sur les produits pétroliers lourds: mesure, interprétation et prévision de la floculation des asphaltènes" REVUE DE L'INSTITUT FRANCAIS DU PÉTROLE, vol. 38, no. 1, January 1983, pages 101-120, XP002016861 ISSN 0020-2274 * page 102, right-hand column, paragraph 1 – page 103, left-hand column, paragraph 3; page 108, right-hand column, paragraph 5 – page 116, right-hand column, paragraph 1; figure 17; table II * | 3-5 | TECHNICAL FIELDS SEARCHED (Int.Cl.6) G01N |

-/--

The present search report has been drawn up for all claims

| Place of search | Date of completion of the search | Examiner |
|---|---|---|
| BERLIN | 2 November 1998 | Johnson, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 98 11 1303

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | ORTIZ-FERNANDEZ M C ET AL: "Regression by least median squares, a methodological contribution to titration analysis" CHEMOMETRICS AND INTELLIGENT LABORATORY SYSTEMS, vol. 27, no. 2, February 1995, page 231-243 XP004037946 ISSN 0169-7439 | | |
| A | FIFIELD FW ET AL: "Principles and practice of analytical chemistry" 1983 , INTERNATIONAL TEXTBOOK COMPANY , LONDON GB XP002082959 * page 225 - page 229 * | | |

TECHNICAL FIELDS SEARCHED     (Int.Cl.6)

The present search report has been drawn up for all claims

| Place of search | Date of completion of the search | Examiner |
|---|---|---|
| BERLIN | 2 November 1998 | Johnson, K |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)